# EUROPÄISCHE PATENTSCHRIFT

(11) **EP 1 989 248 B1**
(45) Veröffentlichungstag und Bekanntmachung des Hinweises auf die Patenterteilung: **05.10.2016**
(21) Anmeldenummer: 07722965.6
(22) Anmeldetag: 28.02.2007
(51) Int. Cl.: C08J 3/00, C08J 3/075, C08F 2/04, C08F 2/44, A61L 15/22

(54) **BIOLOGISCH ABBAUBARE SUPERABSORBIERENDE POLYMERZUSAMMENSETZUNG MIT GUTEN ABSORPTIONS- UND RETENTIONSEIGENSCHAFTEN**
BIODEGRADABLE SUPER-ABSORBENT POLYMER COMPOSITION WITH GOOD ABSORPTION AND RETENTION PROPERTIES
COMPOSITION POLYMÈRE SUPERABSORBANTE BIODÉGRADABLE À BONNES PROPRIÉTÉS D'ABSORPTION ET DE RÉTENTION

(30) Priorität: 28.02.2006 DE 102006009579
(43) Veröffentlichungstag der Anmeldung: 12.11.2008
(73) Patentinhaber: Evonik Degussa GmbH, 45128 Essen (DE)
(72) Erfinder: FURNO, Franck, 40545 Düsseldorf (DE); SCHMIDT, Harald, 47918 Tönisvorst (DE); DE BRUIN, Nicolaas, 47798 Krefeld (DE)
(86) Internationale Anmeldenummer: PCT/EP2007/001712
(87) Internationale Veröffentlichungsnummer: WO 2007/098932

(56) Entgegenhaltungen:
- WO-A-97/27884
- WO-A-2006/101271
- DE-C1- 4 020 780
- GB-A- 2 341 866
- US-A- 4 076 663

## Beschreibung

Die vorliegende Erfindung betrifft allgemein ein Verfahren zur Herstellung einer superabsorbierenden Zusammensetzung, eine superabsorbierende Zusammensetzung erhältlich nach diesem Verfahren, eine teilchenförmige superabsorbierende Zusammensetzung sowie einen Verbund, einen Hygieneartikelcore und einen Hygieneartikel, weiterhin chemische Produkte, wie Schäume, Formkörper, Fasern, Folien, Filme, Kabel, Dichtungsmaterialien, flüssigkeitsaufnehmende Hygieneartikel, Träger für pflanzen- und pilzwachstumsregulierende Mittel, Verpackungsmaterialien, Bodenzusätze oder Baustoffe, jeweils beinhaltend absorbierende Zusammensetzung und auch die Verwendung dieser superabsorbierenden Zusammensetzung in den chemischen Produkten.

Superabsorber sind wasserunlösliche, vernetzte Polymere, die in der Lage sind, unter Quellung und Ausbildung von Hydrogelen große Mengen an wässrigen Flüssigkeiten, insbesondere Körperflüssigkeiten, vorzugsweise Urin oder Blut, aufzunehmen und unter Druck zurückzuhalten. Im Allgemeinen betragen diese Flüssigkeitsaufnahmen das mindestens 10-Fache oder gar das mindestens 100-Fache des Trockengewichts der Superabsorber bzw. der superabsorbierenden Zusammensetzungen an Wasser. Durch diese charakteristischen Eigenschaften finden diese Polymere hauptsächlich Anwendung in Sanitärartikeln wie Babywindeln, Inkontinenzprodukten oder Damenbinden. Einen umfassenden Überblick über Superabsorber bzw. superabsorbierende Zusammensetzungen, ihre Anwendung und ihre Herstellung geben F. L. Buchholz und A. T. Graham (Herausgeber) in "Modern Superabsorbent Polymer Technology", Wiley-VCH, New York, 1998.

Die Herstellung der Superabsorber erfolgt in der Regel durch die radikalische Polymerisation säuregruppen-tragender, meist teilneutralisierter Monomere in Gegenwart von Vernetzern. Dabei lassen sich durch die Auswahl der Monomerzusammensetzung, der Vernetzer sowie der Polymerisationsbedingungen und der Verarbeitungsbedingungen für das nach der Polymerisation erhaltene Hydrogel Polymere mit unterschiedlichen Absorbereigenschaften herstellen.

Auf im Wesentlichen auf Sacchariden wie Zellulose und Stärke basierende absorbierende Polymere sind seit langem bekannt und werden auch heute noch mit dem Ziel die Eignung zum Einsatz in Windeln zu verbessern beforscht, wie sich unter anderem aus WO 2004/085481 A1 ergibt. Um deren Absorptions- und Retentionseigenschaften zu verbessern, wurden derartige Polysaccharide mit Acrylsäure copolymerisiert. Durch diese Copolymerisation wurden zwar die Absorptionsund Retentionseigenschaften derartiger Copolymere verbessert, unter dieser Maßnahme litt jedoch die biologische Abbaubarkeit derartiger Copolymere. In jüngerer Zeit geht der Trend immer mehr dazu, nahezu ausschließlich auf Acrylsäure bzw. Acrylaten basierende Superabsorber herzustellen, da diese schwach vernetzten, teilneutralisierten Polyacrylate im Vergleich zu den Polysacchariden oder den Polysaccharid-Acrylat-Copolymeren die besten Absorptions-, Retentions- und Permeabilitätseigenschaften aufweisen und sich insbesondere in Form von Oberflächen- bzw. nachvernetzten superabsorbierenden Polymeren besonders gut für die Herstellung von hoch mit Superabsorbern gefüllten Sauglagen (mit meist mehr als 50% Superabsorber) eignen, die sich wiederum hervorragend den Hygieneartikeln, insbesondere Damenhygieneartikeln, Babywindeln und Inkontinenzprodukten verarbeiten lassen, um Hygieneartikeln mit hervorragenden Trage- und Gebrauchseigenschaften zu erhalten.

Leider war die biologische Abbaubarkeit dieser nahezu ausschließlich auf Acrylsäure bzw. Acrylaten basierenden superabsorbierenden Polymeren unbefriedigend. Angesichts des steigenden Umweltbewusstseins von Hygieneartikeln nachfragenden Verbrauchern sind die Umweltanforderungen an Hygieneartikel in den letzten Jahren deutlich gestiegen. In ihrer Umweltverträglichkeit verbesserte Hygieneartikel lassen sich jedoch nur dann erfolgreich vermarkten, wenn ihre sonstigen Gebrauchseigenschaften nicht erkennbar gegenüber denen auf herkömmlichen Wegen hergestellten und damit weniger umweltverträglichen Hygieneartikeln zurückstehen.

Allgemein liegt der vorliegenden Erfindung die Aufgabe zugrunde, die sich aus dem Stand der Technik ergebenden Nachteile zu vermindern oder gar zu überwinden.

Es besteht eine erfindungsgemäße Aufgabe darin, durch die Bereitstellung geeigneter Superabsorber herkömmliche Hygieneartikel in ihrer Umweltverträglichkeit zu verbessern, ohne dass diese in ihrer Retentions- bzw. Absorptionsfähigkeit und wenn möglich in ihrer Permeabilität signifikant beeinträchtig werden. Auf diese Weise lässt sich auch bei Cores bzw. Sauglagen mit mehr als 50% Superabsorbergehalt das Phänomen des Gelblocking und des damit verbundenen Leakage lindern oder gar vermeiden.

Zudem liegt eine erfindungsgemäße Aufgabe darin, ein Verfahren zur Verfügung zu stellen, mit dem sich ein Superabsorber schaffen lässt, der die Umweltverträglichkeit von Hygieneartikeln verbessert, ohne ihre Gebrauchsfähigkeit signifikant zu beeinträchtigen.

Ein Beitrag zur Lösung der vorstehenden Aufgaben leisten die Kategorie bildenden Ansprüche und die jeweils nebengeordneten Ansprüche, wobei die von den Kategorie bildenden bzw. Nebenansprüche abhängigen Unteransprüche bevorzugte Ausgestaltungen der vorliegenden Erfindung darstellen.

Einen wichtigen Beitrag zur Lösung mindestens eines Teils dieser Aufgaben leistet ein Verfahren zur Herstellung einer superabsorbierenden Zusammensetzung, beinhaltend als Verfahrensschritte
i. Herstellen eines Hydrogels durch radikalische Polymerisation einer wässrigen, mindestens ein Monomer beinhaltende Monomerlösung;
ii. Trocknen des Hydrogels unter Erhalt eines wasserabsorbierenden Polymergebildes;
iii. Oberflächenvernetzung des wasserabsorbierenden Polymergebildes unter Erhalt eines oberflächenvernetzten wasserabsorbierenden Polymergebildes;
iv. Einarbeiten einer Stärkeverbindung nach Verfahrensschritt i, und vor Verfahrensschritt ii, in das Hydrogel.

Das Verfahren kann auch das Einarbeiten einer Stärkeverbindung nach Verfahrenschritt iii auf das oberflächenvernetzte wasserabsorbierende Polymergebilde umfassen.

Als Stärkeverbindung kommt jede dem Fachmann bekannte und für die vorliegende Erfindung als geeignet erscheinende Stärkeverbindung in Betracht. Unter dem Begriff Stärkeverbindung wird vorliegend sowohl Stärke als solche und gleichfalls Stärkederivate verstanden. Das Einarbeiten der Stärkeverbindung kann iede dem Fachmann bekannten und als geeignet erscheinenden Form erfolgen.

Weiterhin besteht die Möglichkeit, die Stärkeverbindung nach der Oberflächenvernetzung bzw. auf das oberflächenvernetzte wasserabsorbierende Polymergebilde einzuarbeiten. Dieses kann sowohl mit einer angefeuchteten Verbindung, als auch unter Zuhilfenahme eines Bindemittels zum Anhaften der Stärkeverbindung an den oberflächenvernetzten wasserabsorbierenden Polymergebilde oder durch die Kombination von Anfeuchten und Verwendung eines Bindemittels, vorzugsweise eines polymeren Binders, erfolgen. Meist schließt sich an das Aufbringen der Stärkeverbindung ein Reifeschritt an, in dem entweder vorsichtig langsam durchmischt wird oder eine leichte Temperaturbehandlung oder eine Kombination aus diesen beiden Maßnahmen erfolgt.

Im Zusammenhang mit dem erfindungsgemäßen Verfahren ist es weiterhin bevorzugt, dass die darin eingesetzte ein oder mehrere Stärkeverbindung(en) wasserlöslich ist/sind. Stärkeverbindungen gelten insbesondere dann als wasserlöslich, wenn sich bei einer Temperatur von 20°C mindestens 30g der Stärkeverbindung in einem Liter entionisiertem Wasser lösen lassen.

Weiterhin ist es in dem erfindungsgemäßen Verfahren bevorzugt, dass mindestens 70 Gew.-%, vorzugsweise mindestens 80 Gew.-% und besonders bevorzugt mindestens 90 Gew.-% der Stärkeverbindungen eine Teilchengröße von kleiner 100, vorzugsweise kleiner 75 und besonders bevorzugt kleiner 50 µm aufweisen. Ferner ist es bevorzugt, dass die Stärkeverbindungen Gewichtsmittel der Teilchengröße in einem Bereich von 20 bis 80, vorzugsweise von 25 bis 75 und besonders bevorzugt von 30 bis 70 µm besitzen und weniger als 10 Gew.-% der Teilchen eine Teilchengröße von kleiner 5 µm und weniger als 10 Gew.-% eine Teilchengröße von mehr als 90 µm besitzen.

Weiterhin ist es bevorzugt, dass die Stärkeverbindung wenigstens eine, vorzugsweise mindestens zwei und besonders bevorzugt jede der folgenden Eigenschaften besitzt
- S1: eine freie Absorption (FA) von mindestens 9 g/g; besonders bevorzugt mindestens 15 g/g, sowie darüber hinaus bevorzugt mindestens 18 g/g;
- S2: eine Retention (CRC) von mindestens 7 g/g, vorzugsweise mindestens 10 g/g, besonders bevorzugt mindestens 12 g/g und darüber hinaus bevorzugt mindestens 13 g/g, vorzugsweise mindestens 12 g/g;
- S3: eine Absorption gegen Druck (AAP_{0,3}) von mindestens 2 g/g, vorzugsweise mindestens 3 g/g und besonders bevorzugt mindestens 4 g/g, sowie darüber hinaus bevorzugt mindestens 4,5 g/g;
- S4: eine Absorption gegen Druck (AAP_{0,7}) von mindestens 2 g/g, vorzugsweise mindestens 3 g/g, besonders bevorzugt mindestens 4 g/g und darüber hinaus bevorzugt mindestens 5 g/g.

Oftmals erreichen die Absorption, Retention und Absorption gegen Druck der Stärkeverbindungen nicht mehr als 25 bis 35 g/g. Derartige Stärkeverbindungen werden vorzugsweise in der Variante V1 eingesetzt.

Weiterhin ist es in den erfindungsgemäßen Verfahren bevorzugt, dass mindestens eine erste Stärkeverbindung SI nach Verfahrensschritt i, und vor Verfahrensschritt ii, in das Hydrogel eingearbeitet wird. Als Stärkeverbindung SI sind insbesondere auf Stärke basierenden Verbindungen bevorzugt, die einen geringen Amylosegehalt, d.h. weniger als 60%, vorzugsweise weniger als 50% und darüber hinaus bevorzugt weniger als 30%, jeweils bezogen auf die gesamte Stärkeverbindung, Amylose aufweisen. Weiterhin basieren Stärkeverbindungen SI vorzugsweise auf Kartoffelstärke. Darüber hinaus ist es bevorzugt, dass Stärkeverbindungen SI mindestens teilweise carboxyalkyliert sind. Als Alkylverbindungen kommen hier insbesondere niedere Alkylverbindungen wie Methyl, Ethyl, Propyl und Butyl in Betracht, wobei Methyl und Ethyl bevorzugt und Methyl besonders bevorzugt sind. Vorzugsweise ist die Stärkeverbindung SI zu mindestens 10, vorzugsweise mindestens 50 und darüber hinaus mindestens 75%, jeweils bezogen auf die Stärkeverbindung SI, carboxyalkyliert. Bevorzugte Stärkeverbindungen SI basieren auf Kartoffelstärke, enthalten weniger als 20% Amylose und sind zu mehr als 75% carboxymethyliert.

Zudem ist es in dem erfindungsgemäßen Verfahren bevorzugt, dass zusätzlich zu der ersten Stärkeverbindung SI mindestens eine weitere Stärkeverbindung SII nach Verfahrensschritt iii auf das oberflächenvernetzte wasserabsorbierende Polymergebilde eingearbeitet wird. Vorzugsweise wird diese mindestens eine weitere Stärkeverbindung SII in Variante 5, d.h. in dem Verfahrensschritt iii., der Oberflächenvernetzung, eingearbeitet. Es ist ferner bevorzugt, dass in dem erfindungsgemäßen Verfahren sich die erste Stärkeverbindung SI und die weitere Stärkeverbindung SII in mindestens einem Merkmal voneinander unterscheiden. Bevorzugt ist, dass die erste Stärkeverbindung SI und die weitere Stärkeverbindung SII in mindestens einem, vorzugsweise mindestens zwei und darüber hinaus bevorzugt jedem der folgenden Merkmale voneinander unterscheiden:
- D1: in der freien Absorption (FA), hierbei vorzugsweise um eine Differenz von mindestens 0,1, vorzugsweise mindestens 1 und besonders bevorzugt mindestens 5 und darüber hinaus bevorzugt mindestens 15 g/g;
- D2: in der Retention (CRC), hierbei vorzugsweise um eine Differenz von mindestens 0,1, vorzugsweise mindestens 1 und besonders bevorzugt mindestens 3 und darüber hinaus bevorzugt mindestens 6 g/g;
- D3: in dem pH-Wert, hierbei vorzugsweise um eine Differenz von mindestens 0,1, vorzugsweise mindestens 1 und besonders bevorzugt mindestens 3 und darüber hinaus bevorzugt mindestens 6;
- D4: in der chemischen Zusammensetzung, vorzugsweise in dem Grad der carboxyalkylierung oder des Amylosegehalts und besonders bevorzugt in dem Grad der carboxyalkylierung und des Amylosegehalts.

Vorzugsweise unterscheiden sich die Stärkeverbindungen SI und SII in den Merkmalen D1 bis D3.

Außerdem ist es in dem erfindungsgemäßen Verfahren bevorzugt, dass die Stärkeverbindung SII mindestens eine der folgenden Eigenschaften besitzt:
- d1: freie Absorption (FA) von weniger als 9 g/g, vorzugsweise weniger als 8 g/g und bevorzugt weniger als 6 g/g;
- d2: eine Retention (CRR) von weniger als 7 g/g, vorzugsweise weniger als 6 g/g und besonders bevorzugt weniger als 5,5 g/g;
- d3: einen pH-Wert im Bereich von 3 bis < 9, vorzugsweise 4 bis 8,5 und besonders bevorzugt 5 bis 8.

Besonders bevorzugt ist es, dass die Stärkeverbindung SII mindestens die Merkmale d1 oder d2 jeweils mit d3 besitzt.

Weiterhin ist es bevorzugt, dass die Stärkeverbindung SII im Vergleich zur Stärkeverbindung SI einen größeren Amylosegehalt aufweist. So ist es bevorzugt, dass die Stärkeverbindung SII mindestens 60, bevorzugt mindestens 65 und darüber hinaus mindestens 70% Amylose aufweist. Es entspricht weiterhin einer Ausgestaltung des erfindungsgemäßen Verfahrens, dass die Stärkeverbindung SII auch für sich, d.h. als einzige Stärkeverbindung, eingesetzt werden kann.

Im allgemeinen können die Stärkeverbindungen in dem erfindungsgemäßen Verfahren in einer Menge von max. 30 Gew.-%, vorzugsweise maximal 20 Gew.-%, darüber hinaus bevorzugt maximal 15 Gew.-%, sowie weiterhin bevorzugt maximal 10 Gew.-%, jeweils bezogen auf das eingesetzte Monomer, eingesetzt werden. Für den Fall, dass mehr als eine Stärkeverbindung, insbesondere eine erste Stärkeverbindung SI und mindestens eine weitere Stärkeverbindung SII eingesetzt werden, behalten die vorstehend erfolgten maximalen Stärkeverbindungsmengen ebenfalls ihre Gültigkeit, wobei diese sich dann auf alle Stärkeverbindungen beziehen.

Zudem ist es in dem erfindungsgemäßen Verfahren bevorzugt, dass die Monomerlösung einen Gehalt an Monomer im Bereich von 15 bis 35 Gew.-%, vorzugsweise im Bereich von 20 bis 30 Gew.-% sowie darüber hinaus bevorzugt in einem Bereich von 22,5 bis 27,5 Gew.-%, jeweils bezogen auf die Monomerlösung, hat.

Weiterhin ist es in dem erfindungsgemäßen Verfahren bevorzugt, dass die Stärkeverbindung eine Lösungsviskosität nach der hier angegebenen Methode in einem Bereich von 5000 bis 300000 mPas, vorzugsweise in einem Bereich von 10000 bis 100000 mPas und darüber hinaus bevorzugt in einem Bereich von 20000 bis 50000 mPas hat.

Weiterhin ist in dem erfindungsgemäßen Verfahren bevorzugt, dass die Stärkeverbindung weniger als 10 Gew.-%, vorzugsweise weniger als 7 Gew.-% und darüber hinaus bevorzugt weniger als 3 Gew.-% zyklische oder verzweigte Polysaccharide aufweist.

Geeignete Stärkeverbindungen sind die von der Firma Roquette GmbH, Deutschland, unter den Bezeichnungen Tackidex^{®}, zum Beispiel Tackidex^{®} 009 oder Tackidex^{®} Q 106, Eurylon^{®}, zum Beispiel Eurylon^{®} 7, Floralys^{®}, zum Beispiel Floralys^{®} 380, oder die Produkte T 547, T 548 oder T 549.

Ferner ist es nach dem erfindungsgemäßen Verfahren bevorzugt, dass die superabsorbierende Zusammensetzung mindestens eine, vorzugsweise mindestens zwei und darüber hinaus bevorzugt jede der folgenden Eigenschaften besitzt.
- Z1: Eine Retention (CRC) von mindestens 25 g/g, vorzugsweise mindestens 27 g/g und besonders bevorzugt mindestens 29 g/g;
- Z2: eine Absorption gegen Druck (AAP_{0,7}) von mindestens 15 g/g, vorzugsweise mindestens 17 g/g und besonders bevorzugt mindestens 19 g/g;
- Z3: eine Permeabilität (SFC) von mindestens 20*10⁻⁷ cm³sec/g, vorzugsweise mindestens 24*10⁻⁷ cm³sec/g und besonders bevorzugt mindestens 28*10⁻⁷ cm³ sec/g;
- Z4: eine gemäß dem hier angegebenen Test bestimmte biologische Abbaubarkeit nach 28 Tagen von mindestens 25%, vorzugsweise mindestens 35 % und am meisten bevorzugt mindestens 45 %.

Alle hier möglichen Kombinationen der Ziffern Z1 bis Z4 stellen bevorzugte Ausführungsformen des erfindungsgemäßen Verfahrens dar, wobei Kombination Z1Z2, Z1Z3, Z1Z4, Z1Z2Z4, Z1Z2Z3 und Z2Z3Z4 bevorzugt sind, wobei die Kombination mit Z4 besonders bevorzugt ist. Hierunter fallen Z4Z1, Z4Z2 und Z4Z3, wobei Z4Z1 und Z4Z2 besonders bevorzugte Ausgestaltungen der Eigenschaften der durch das erfindungsgemäße Verfahren erhältlichen superabsorbierenden Zusammensetzungen darstellen. Oftmals werden Absorption gegen Druck Werte von 30 bis 50 g/g und Retentionswerte von 50 bis 70 g/g sowie Permeabilitätswerte von 90*10⁻⁷ cm³sec/g bis 110*10⁻⁷ cm³sec/g als Maximalwerte nicht überschritten, wobei auch ein Wert bei der biologischen Abbaubarkeit von maximal 75 bis 95 % als Obergrenze im oftmals nicht überschritten wird.

Nach einer anderen Ausgestaltung der erfindungsgemäßen superabsorbierenden Zusammensetzung und des Verfahrens zu deren Herstellung ist es bevorzugt, dass dazu eine von dem wasserabsorbierenden Polymergebilde verschiedener Bindemittel bzw. polymerer Binder eingesetzt wird, den die erfindungsgemäß hergestellte Zusammensetzung beinhaltet. Als polymere Binder kommen grundsätzlich alle dem Fachmann für den Zweck der Fixierung der Stärkeverbindung bekannten und geeignet erscheinenden polymeren Binder in Betracht. Unter diesen sind thermoplastische Polymere bevorzugt. In diesem Zusammenhang wird auf die in WO 2005/011860 beschriebenen thermoplastischen Polymere Bezug genommen. Vorzugsweise weisen derartige polymere Binder ein durch GPC (Gelpermeationschromatographie) bestimmtes und mittels Lichtstreuung absolut detektierbares Molekulargewicht in einem Bereich von 1000 bis 100.000, vorzugsweise 2000 bis 50.000 und darüber hinaus bevorzugt 3000 bis 15.000 g/Mol auf. Weiterhin ist es bevorzugt, dass derartige polymere Binder zwei oder mehr OH-Gruppen beinhalten. Beispiele geeigneter polymerer Binder sind Polyalkylenglykole, wie Polyethylenglykol, Polypropylenglykol, wobei Polyethylenglykol und insbesondere ein Polyethylenglykol mit einem Molekulargewicht in einem Bereich von 5000 bis 15.000 g/mol, besonders bevorzugt ist. Die hier angegebenen Molekulargewichte betreffen jeweils das Gewichtsmittel des Molekulargewichts.

Erfindungsgemäß bevorzugte Polymergebilde sind Fasern, Schäume oder Teilchen, wobei Fasern und Teilchen bevorzugt und Teilchen besonders bevorzugt sind. Gleiches gilt auch für die erfindungsgemäße superabsorbierende Zusammensetzung.

Erfindungsgemäß bevorzugte Polymerfasern sind so dimensioniert, dass sie in oder als Garne für Textilien und auch direkt in Textilien eingearbeitet werden können. Es ist erfindungsgemäß bevorzugt, dass die als Polymerfasern vorliegenden Polymergebilde eine Länge in einem Bereich von 1 bis 500 mm, bevorzugt 2 bis 500 mm und besonders bevorzugt 5 bis 100 mm und einen Durchmesser in einem Bereich von 1 bis 200 Denier, bevorzugt 3 bis 100 Denier und besonders bevorzugt 5 bis 60 Denier besitzen.

Erfindungsgemäß bevorzugte Polymerteilchen sind so dimensioniert, dass sie eine mittlere Teilchengröße gemäß ERT 420.2-02 in einem Bereich von 10 bis 3000 µm, vorzugsweise 20 bis 2000 µm und besonders bevorzugt 150 bis 850 µm aufweisen. Dabei ist es insbesondere bevorzugt, dass der Anteil der Polymerteilchen mit einer Partikelgröße in einem Bereich von 300 bis 600 µm mindestens 30 Gew.-%, besonders bevorzugt mindestens 40 Gew.-% und am meisten bevorzugt mindestens 50 Gew.-%, bezogen auf das Gesamtgewicht der nachvernetzten, wasserabsorbierenden Polymerteilchen, beträgt.

In einer bevorzugten Ausführungsform der erfindungsgemäß eingesetzten wasserabsorbierenden Polymergebilde basieren diese auf
(α1) 20-99,999 Gew.-%, bevorzugt 55-98,99 Gew.-% und besonders bevorzugt 70-98,79 Gew.-% polymerisierten, ethylenisch ungesättigten, säuregruppen-tragenden Monomeren oder deren Salze oder polymerisierten, ethylenisch ungesättigten, einen protonierten oder quarternierten Stickstoff beinhaltenden Monomeren, oder deren Mischungen, wobei mindestens ethylenisch ungesättigte, säuregruppenhaltige Monomere, vorzugsweise Acrylsäure, beinhaltende Mischungen besonders bevorzugt sind,
(α2) 0-80 Gew.-%, vorzugsweise 0-44,99 Gew.-% und besonders bevorzugt 0,1-44,89 Gew.-% polymerisierten, monoethylenisch ungesättigten, mit (α1) copolymerisierbaren Monomeren,
(α3) 0,001-5 Gew.-%, vorzugsweise 0,01-3 Gew.-% und besonders bevorzugt 0,01-2,5 Gew.-% eines oder mehrerer Vernetzer,
(α4) 0 bis 30 Gew.-%, vorzugsweise 0 bis 5 Gew.-% und besonders bevorzugt 0,1-5 Gew.-% eines wasserlöslichen Polymeren,
(α5) 0 bis 20 Gew.-%, vorzugsweise 2,5 bis 15 Gew.-% und besonders bevorzugt 3 bis 10 Gew.-% Wasser, sowie
(α6) 0 bis 20 Gew.-%, vorzugsweise 0 bis 10 Gew.-% und besonders bevorzugt 0,1 bis 8 Gew.-% eines oder mehrerer Hilfsmittel, wobei die Summe der Gewichtsmengen (α1) bis (α6) 100 Gew.-% beträgt.

Die monoethylenisch ungesättigten, säuregruppenhaltigen Monomere (α1) können teilweise oder vollständig, bevorzugt teilweise neutralisiert sein. Vorzugsweise sind die monoethylenisch ungesättigten, säuregruppenhaltigen Monomere zu mindestens 25 Mol-%, besonders bevorzugt zu mindestens 50 Mol-% und darüber hinaus bevorzugt zu 50-80 Mol-% neutralisiert. In diesem Zusammenhang wird auf DE 195 29 348 A1 verwiesen. Die Neutralisation kann teilweise oder ganz auch nach der Polymerisation erfolgen. Ferner kann die Neutralisation mit Alkalimetallhydroxiden, Erdalkalimetallhydroxiden, Ammoniak sowie Carbonaten und Bicarbonaten erfolgen. Daneben ist jede weitere Base denkbar, die mit der Säure ein wasserlösliches Salz bildet. Auch eine Mischneutralisation mit verschiedenen Basen ist denkbar. Bevorzugt ist die Neutralisation mit Ammoniak und Alkalimetallhydroxiden, besonders bevorzugt mit Natriumhydroxid und mit Ammoniak.

Ferner können bei einem Polymer die freien Säuregruppen überwiegen, so dass dieses Polymer einen im sauren Bereich liegenden pH-Wert aufweist. Dieses saure wasserabsorbierende Polymer kann durch ein Polymer mit freien basischen Gruppen, vorzugsweise Amingruppen, das im Vergleich zu dem sauren Polymer basisch ist, mindestens teilweise neutralisiert werden. Diese Polymere werden in der Literatur als "Mixed-Bed Ion-Exchange Absorbent Polymers" (MBIEA-Polymere) bezeichnet und sind unter anderem in der WO 99/34843 A1 offenbart. In der Regel stellen MBIEA-Polymere eine Zusammensetzung dar, die zum einen basische Polymere, die in der Lage sind, Anionen auszutauschen, und andererseits ein im Vergleich zu dem basischen Polymer saures Polymer, das in der Lage ist, Kationen auszutauschen, beinhalten. Das basische Polymer weist basische Gruppen auf und wird typischerweise durch die Polymerisation von Monomeren erhalten, die basische Gruppen oder Gruppen tragen, die in basische Gruppen umgewandelt werden können. Bei diesen Monomeren handelt es sich vor allen Dingen um solche, die primäre, sekundäre oder tertiäre Amine oder die entsprechenden Phosphine oder mindestens zwei der vorstehenden funktionellen Gruppen aufweisen. Zu dieser Gruppe von Monomeren gehören insbesondere Ethylenamin, Allylamin, Diallylamin, 4-Aminobuten, Alkyloxycycline, Vinylformamid, 5-Aminopenten, Carbodiimid, Formaldacin, Melamin und dergleichen, sowie deren sekundäre oder tertiäre Aminderivate.

Bevorzugte ethylenisch ungesättigte, säuregruppenhaltige Monomere (α1) sind vorzugsweise diejenigen Verbindungen, die in der WO 2004/037903 A2, als Referenz eingeführt wird und somit als Teil der Offenbarung gilt, als ethylenisch ungesättigte, säuregruppenhaltige Monomere (α1) genannt werden. Besonders bevorzugte ethylenisch ungesättigte, säuregruppenhaltige Monomere (α1) sind Acrylsäure und Methacrylsäure, wobei Acrylsäure am meisten bevorzugt ist.

Gemäß einer Ausführungsform der vorliegenden Erfindung werden wasserabsorbierende Polymergebilde eingesetzt, bei denen die monoethylenisch ungesättigten, mit (α1) copolymerisierbare Monomere (α2) Acrylamide, Methacrylamide oder Vinylamide sind.

Bevorzugte (Meth)acrylamide sind neben Acrylamid und Methacrylamid alkylsubstituierte (Meth)acrylamide oder aminoalkylsubstituierte Derivate des (Meth)acrylamids, wie N-Methylol(meth)acrylamid, N,N-Dimethylamino-(meth)acrylamid, Dimethyl(meth)acrylamid oder Diethyl(meth)acrylamid. Mögliche Vinylamide sind beispielsweise N-Vinylamide, N-Vinylformamide, N-Vinylacetamide, N-Vinyl-N-Methylacetamide, N-Vinyl-N-methylformamide, Vinylpyrrolidon. Unter diesen Monomeren besonders bevorzugt ist Acrylamid.

Gemäß einer anderen Ausführungsform der vorliegenden Erfindung werden wasserabsorbierende Polymergebilde eingesetzt, bei denen die monoethylenisch ungesättigten, mit (α1) copolymerisierbaren Monomere (α2) wasserlösliche Monomere sind. In diesem Zusammenhang sind insbesondere Alkoxypolyalkylenoxid(meth)acrylate wie Methoxypolyethylenglykol(meth)acrylate bevorzugt.

Des Weiteren sind als monoethylenisch ungesättigte, mit (α1) copolymerisierbaren Monomere (α2) in Wasser dispergierbare Monomere bevorzugt. Als in Wasser dispergierbare Monomere sind Acrylsäureester und Methacrylsäureester, wie Methyl(meth)acrylat, Ethyl(meth)acrylat, Propyl(meth)acrylat oder Butyl(meth)-acrylat.

Die monoethylenisch ungesättigten, mit (α1) copolymerisierbaren Monomere (α2) umfassen weiterhin Methylpolyethylenglykolallylether, Vinylacetat, Styrol und Isobutylen.

Als Vernetzer (α3) werden vorzugsweise diejenigen Verbindungen eingesetzt, die in der WO 2004/037903 A2 als Vernetzer (α3) genannt werden. Unter diesen Vernetzern sind wasserlösliche Vernetzer besonders bevorzugt. Am meisten bevorzugt sind dabei N,N'-Methylenbisacrylamid, Polyethylenglykol-di(meth)acrylate, Triallylmethylammoniumchlorid, Tetraallylammoniumchlorid sowie mit 9 Mol Ethylenoxid pro Mol Acrylsäure hergestelltes Allylnonaethylenglykolacrylat besonders bevorzugt.

Als wasserlösliche Polymere (α4) können in den Polymergebilden wasserlösliche Polymerisate, wie teil- oder vollverseifter Polyvinylalkohol, Polyvinylpyrrolidon, Polyglykole oder Polyacrylsäure enthalten, vorzugsweise einpolymerisiert sein. Das Molekulargewicht dieser Polymere ist unkritisch, solange sie wasserlöslich sind. Bevorzugte wasserlösliche Polymere sind Polyvinylalkohole. Die wasserlöslichen Polymere, vorzugsweise synthetische wie Polyvinylalkohole, können auch als Pfropfgrundlage für die zu polymerisierenden Monomeren dienen.

Als Hilfsmittel (α6) sind vorzugsweise Stellmittel, Geruchsbinder, oberflächenaktive Mittel oder Antioxidatien sowie diejenigen Additive, die zur Herstellung der Polymergebilde eingesetzt wurden (Initiatoren usw.) in den Polymergebilden enthalten.

Als wasserabsorbierende Polymergebilde werden erfindungsgemäße, vorzugsweise Polymere eingesetzt, welche erhalten wurden durch ein Verfahren umfassend die Verfahrensschritte:
a) radikalische Polymerisation säuregruppen-tragender, ethylenisch ungesättigter, gegebenenfalls teilneutralisierter Monomere in Gegenwart eines Vernetzers unter Bildung eines Hydrogels;
b) gegebenenfalls Zerkleinern des Hydrogels;
c) Trocknen des gegebenenfalls zerkleinerten Hydrogels unter Erhalt wasserabsorbierender Polymergebilde;
d) gegebenenfalls Zermahlen des so erhaltenen absorbierenden Polymergebildes und Absieben auf eine gewünschte Partikelgrößenfraktion;
e) gegebenenfalls weitere Oberflächenmodifizierungen der so erhaltenen wasserabsorbierenden Polymergebilde

Geeignete Mischaggregate zur Oberflächenmodifizierung und auch zum Aufbringen des Geruchsbinders oder zumindest einer seiner Komponenten sind der Patterson-Kelley-Mischer, DRAIS-Turbulenzmischer, Lödigemischer, Ruberg-Mischer, Schneckenmischer, Tellermischer und Wirbelschichtmischer sowie kontinuierlich arbeitende senkrechte Mischer, in denen das Polymergebilde mittels rotierender Messer in schneller Frequenz gemischt wird (Schugi-Mischer).

Die im Verfahrensschritt a) erfolgende radikalische Polymerisation erfolgt vorzugsweise in wässriger Lösung, wobei diese wässrige Lösung neben Wasser als Lösungsmittel vorzugsweise
(α1) die ethylenisch ungesättigten, säuregruppen-tragenden Monomeren oder deren Salze, wobei Acrylsäure als säuregruppen-tragendes Monomer besonders bevorzugt ist,
(α2) gegebenenfalls monoethylenisch ungesättigte, mit (α1) copolymerisierbare Monomere,
(α3) den Vernetzer,
(α4) gegebenenfalls ein wasserlösliches Polymer, sowie
(α6) gegebenenfalls ein oder mehrere Hilfsmittel

### beinhaltet.

Als ethylenisch ungesättigte, säuregruppen-tragenden Monomere (α1), als monoethylenisch ungesättigte, mit (α1) copolymerisierbare Monomere (α2), als Vernetzer (α3), als wasserlösliche Polymere (α4) und als Hilfsmittel (α6) sind wiederum diejenigen Verbindungen bevorzugt, die bereits eingangs im Zusammenhang mit den erfindungsgemäßen Polymergebilden als ethylenisch ungesättigte, säuregruppen-tragenden Monomere (α1), als monoethylenisch ungesättigte, mit (α1) copolymerisierbare Monomere (α2), als Vernetzer (α3), als wasserlösliche Polymere (α4) und als Hilfsmittel (α6) genannt wurden.

Aus den vorgenannten Monomeren, Comonomeren, Vernetzern, wasserlöslichen Polymeren und Hilfsstoffen lassen sich die wasserabsorbierende Polymergebilde durch verschiedene Polymerisationsweisen herstellen. Beispielsweise sind in diesem Zusammenhang Massepolymerisation, die vorzugsweise in Knetreaktoren wie Extrudern erfolgt, Lösungspolymerisation, Spraypolymerisation, inverse Emulsionspolymerisation und inverse Suspensionspolymerisation zu nennen.

Bevorzugt wird die Lösungspolymerisation in Wasser als Lösungsmittel durchgeführt. Die Lösungspolymerisation kann kontinuierlich durch Polymerisation auf einem die Reaktionsmischung weiterfördernden Band, wie in DE 35 44 770 A1 offenbart, oder diskontinuierlich erfolgen. Aus dem Stand der Technik ist ein breites Spektrum von Variationsmöglichkeiten hinsichtlich der Reaktionsverhältnisse wie Temperaturen, Art und Menge der Initiatoren als auch der Reaktionslösung zu entnehmen. Typische Verfahren sind in den folgenden Patentschriften beschrieben: US 4,286,082, DE 27 06 135, US 4,076,663, DE 35 03 458, DE 35 44 770, DE 40 20 780, DE 42 44 548, DE 43 23 001, DE 43 33 056, DE 44 18 818.

Die Polymerisation wird wie allgemein üblich durch einen Initiator ausgelöst. Als Initiatoren zur Initiierung der Polymerisation können alle unter den Polymerisationsbedingungen Radikale bildende Initiatoren verwendet werden, die üblicherweise bei der Herstellung von Superabsorbern eingesetzt werden. Auch eine Initiierung der Polymerisation durch Einwirkung von Elektronenstrahlen auf die polymerisierbare, wässrige Mischung ist möglich. Die Polymerisation kann allerdings auch in Abwesenheit von Initiatoren der oben genannten Art durch Einwirkung energiereicher Strahlung in Gegenwart von Photoinitiatoren ausgelöst werden. Polymerisationsinitiatoren können in einer Lösung erfindungsgemäßer Monomere gelöst oder dispergiert enthalten sein. Als Initiatoren kommen sämtliche dem Fachmann bekannten in Radikale zerfallenden Verbindungen in Betracht. Hierunter fallen insbesondere diejenigen Initiatoren, die bereits in der WO 2004/037903 A2 als mögliche Initiatoren genannt werden.

Besonders bevorzugt wird zur Herstellung der wasserabsorbierenden Polymergebilde ein Redoxsystem bestehend aus Wasserstoffperoxid, Natriumperoxodisulfat und Ascorbinsäure eingesetzt.

Auch die inverse Suspensions- und Emulsionspolymerisation kann zur Herstellung der Polymergebilde angewendet werden. Gemäß diesen Prozessen wird eine wässrige, teilneutralisierte Lösung der Monomere (α1), und (α2), gegebenenfalls beinhaltend wasserlösliche Polymere und Hilfsstoffe, mit Hilfe von Schutzkolloiden und/oder Emulgatoren in einem hydrophoben, organischen Lösungsmittel dispergiert und durch Radikalinitiatoren die Polymerisation gestartet. Die Vernetzer sind entweder in der Monomerlösung gelöst und werden mit dieser zusammen dosiert oder aber separat und gegebenenfalls während der Polymerisation zugefügt. Gegebenenfalls erfolgt die Zugabe eines wasserlöslichen Polymeren (α4) als Pfropfgrundlage über die Monomerlösung oder durch direkte Vorlage in die Ölphase. Anschließend wird das Wasser azeotrop aus dem Gemisch entfernt und das Polymerisat abfiltriert.

Weiterhin kann sowohl bei der Lösungspolymerisation als auch bei der inversen Suspensions- und Emulsionspolymerisation die Vernetzung durch Einpolymerisation des in der Monomerlösung gelösten polyfunktionellen Vernetzers und/oder durch Reaktion geeigneter Vernetzer mit funktionellen Gruppen des Polymeren während der Polymerisationsschritte erfolgen. Die Verfahren sind beispielsweise in den Veröffentlichungen US 4,340,706, DE 37 13 601, DE 28 40 010 und WO 96/05234 A1 beschrieben.

Die bei der Lösungspolymerisation oder der inversen Suspensions- und Emulsionspolymerisation im Verfahrensschritt a) erhaltenen Hydrogele werden im Verfahrensschritt c) getrocknet.

Insbesondere im Falle der Lösungspolymerisation ist es jedoch bevorzugt, dass die Hydrogele vor der Trocknung zunächst in einem zusätzlichen Verfahrensschritt b) zerkleinert werden. Dieses Zerkleinern erfolgt durch dem Fachmann bekannte Zerkleinerungsvorrichtungen, wie ein Hackmesser (vgl. DE 195 18 645 C1) oder etwa einen Fleischwolf, der dem Hackmesser nachgeschaltet sein kann.

Die Trocknung des Hydrogels erfolgt vorzugsweise in geeigneten Trocknern oder Öfen. Beispielhaft seien Drehrohröfen, Wirbelbetttrockner, Tellertrockner, Paddeltrockner oder Infrarottrockner genannt. Weiterhin ist es erfindungsgemäß bevorzugt, dass die Trocknung des Hydrogels im Verfahrensschritt c) bis zu einem Wassergehalt von 0,5 bis 25 Gew.-%, vorzugsweise von 1 bis 10 Gew.-% erfolgt, wobei die Trocknungstemperaturen üblicherweise in einem Bereich von 100 bis 200°C liegen.

Die im Verfahrensschritt c) erhaltenen, getrockneten wasserabsorbierenden Polymergebilde können, insbesondere dann, wenn sie durch Lösungspolymerisation erhalten wurden, in einem weiteren Verfahrensschritt d) noch zermahlen und auf die eingangs genannte Wunschkorngröße abgesiebt werden. Das Zermahlen der getrockneten, wasserabsorbierenden Polymergebilde erfolgt vorzugsweise in geeigneten, mechanischen Zerkleinerungsvorrichtungen, wie etwa einer Kugelmühle.

Im Anschluss an die Trocknung der Hydrogele und nach der gegebenenfalls erfolgten weiteren Konfektionierung der getrockneten wasserabsorbierenden Polymergebilde können diese in einem weiteren Verfahrenschritt e) im Oberflächenbereich modifiziert werden. Dieses kann durch ein Metallsalz oder mit der Kombination aus dem Metallsalz und dem Oxid eines Metalls oder einem andere Modifizierungsmittel oder durch ein anderes Modifizierungsmittel erfolgen, wobei die vorstehenden Stoffe als wässrige Lösung oder als Feststoff aufgetragen werden können. Die Konfektionierung kann sowohl auf dem wasserabsorbierenden Polymergebilde als auch auf der den Geruchsbinder bereits beinhaltenden superabsorbierenden Zusammensetzung erfolgen.

Als bevorzugte Modifizierungsmaßnahme sei hier die Oberflächennachvernetzung genannt, bei der die getrockneten Polymergebilde oder aber das noch nicht getrocknete, jedoch vorzugsweise bereits zerkleinerte Hydrogel mit einem vorzugsweise organischen, chemischen Oberflächennachvernetzer in Kontakt gebracht wird. Dabei wird der Nachvernetzer insbesondere dann, wenn er unter den Nachvernetzungsbedingungen nicht flüssig ist, über ein Lösungsmittel mit den Polymerteilchen bzw. dem Hydrogel in Kontakt gebracht. Als Lösungsmittel werden dabei vorzugsweise Wasser, mit Wasser mischbare organische Lösungsmittel wie etwa Methanol, Ethanol, 1-Propanol, 2-Propanol oder 1-Butanol oder Mischungen aus mindestens zwei dieser Lösungsmittel eingesetzt, wobei Wasser als Lösungsmittel am meisten bevorzugt ist. Weiterhin ist es bevorzugt, dass der Nachvernetzer in dem Lösungsmittel oder -gemisch in einer Menge in einem Bereich von 5 bis 75 Gew.-%, besonders bevorzugt 10 bis 50 Gew.-% und am meisten bevorzugt 15 bis 40 Gew.-%, bezogen auf das Gesamtgewicht des Lösungsmittels oder -gemisches, enthalten ist.

Das Inkontaktbringen des Polymergebildes bzw. des zerkleinerten Hydrogels mit dem Lösungsmittel oder -gemisch beinhaltend den Nachvernetzer erfolgt im erfindungsgemäßen Verfahren vorzugsweise durch gutes Vermischen des Lösungsmittels oder -gemischs mit dem Polymergebilde.

Geeignete Mischaggregate zum Vermischen sind z. B. der Patterson-Kelley-Mischer, DRAIS-Turbulenzmischer, Lödigemischer, Ruberg-Mischer, Schneckenmischer, Tellermischer und Wirbelschichtmischer sowie kontinuierlich arbeitende senkrechte Mischer, in denen das Polymergebilde mittels rotierender Messer in schneller Frequenz gemischt wird (Schugi-Mischer).

Das Polymergebilde wird in dem erfindungsgemäßen Verfahren bei der Nachvernetzung vorzugsweise mit höchstens 20 Gew. -%, besonders bevorzugt mit höchstens 15 Gew.-%, darüber hinaus bevorzugt mit höchstens 10 Gew. -%, darüber hinaus noch mehr bevorzugt mit höchstens 5 Gew.-% an Lösungsmittel, vorzugsweise Wasser, in Kontakt gebracht.

Bei Polymergebilden in der Form von vorzugsweise näherungsweise kugelförmigen Teilchen ist es erfindungsgemäß weiterhin bevorzugt, dass das in Kontakt bringen derart erfolgt, dass lediglich der Außenbereich, nicht jedoch der innere Bereich der teilchenförmigen Polymergebilde mit dem Lösungsmittel oder -gemisch und somit dem Nachvernetzer in Kontakt gebracht werden.

Als Nachvernetzer, die im erfindungsgemäßen Verfahren eingesetzt werden, werden vorzugsweise Verbindungen verstanden, die mindestens zwei funktionelle Gruppen aufweisen, die mit funktionellen Gruppen eines Polymergebildes in einer Kondensationsreaktion (=Kondensationsvernetzer), in einer Additionsreaktion oder in einer Ringöffnungsreaktion reagieren können. Als Nachvernetzer sind im erfindungsgemäßen Verfahren diejenigen bevorzugt, die in WO 2004/037903 A2 als Vernetzer der Vernetzerklassen II genannt wurden.

Unter diesen Verbindungen sind als Nachvernetzer besonders bevorzugt Kondensationsvernetzer wie beispielsweise Diethylenglykol, Triethylenglykol, Polyethylenglykol, Glyzerin, Polyglyzerin, Propylenglykol, Diethanolamin, Triethanolamin, Polyoxypropylen, Oxyethylen-Oxypropylen-Blockcopolymere, Sorbitanfettsäureester, Polyoxyethylensorbitanfettsäureester, Trimethylolpropan, Pentaerythrit, Polyvinylalkohol, Sorbitol, 1,3-Dioxolan-2-on (Ethylencarbonat), 4-Methyl-1,3-dioxolan-2-on (Propylencarbonat), 4,5-Dimethyl-1,3-dioxolan-2-on, 4,4-Dimethyl-1,3-dioxolan-2-on, 4-Ethyl-1,3-dioxolan-2-on, 4-Hydroxymethyl-1, 3-dioxolan-2-on, 1,3-Dioxan-2-on, 4-Methyl-1,3-dioxan-2-on, 4,6-Dimethyl-1,3-dioxan-2-on sowie besonders bevorzugt 1,3-Dioxolan-2-on.

Nachdem die Polymergebilde bzw. die Hydrogele mit dem Nachvernetzer bzw. mit dem Fluid beinhaltend den Nachvernetzer in Kontakt gebracht wurden, werden sie auf eine Temperatur in einem Bereich von 50 bis 300°C, vorzugsweise 75 bis 275°C und besonders bevorzugt 150 bis 250°C erhitzt, so dass vorzugsweise der Aussenbereich der Polymergebilde im Vergleich zum Innenbereich stärker vernetzt wird (=Nachvernetzung). Die Zeitdauer der Wärmebehandlung wird durch die Gefahr, dass das gewünschte Eigenschaftsprofil der Polymergebilde infolge von Hitzeeinwirkung zerstört wird, begrenzt.

Ferner leistet eine superabsorbierende Zusammensetzung, die nach dem erfindungsgemäßen Verfahren erhältlich ist einen Beitrag zur Lösung mindestens einer Aufgabe der vorliegenden Erfindung. Dieses gilt insbesondere dann, wenn diese Zusammensetzung die Z-Eigenschaften (Z1 bis Z4) in den ebenfalls vorstehend beschriebenen Variationen aufweist.

Einen weiteren Beitrag zur Lösung der eingangs beschriebenen Aufgaben liefert ein Verbund, beinhaltend die erfindungsgemäße superabsorbierende Zusammensetzung und ein Substrat. Es ist dabei bevorzugt, dass die erfindungsgemäßen superabsorbierende Zusammensetzung und das Substrat miteinander fest verbunden sind. Als Substrate sind Folien aus Polymeren, wie beispielsweise aus Polyethylen, Polypropylen oder Polyamid, Metalle, Vliese, Fluff, Tissues, Gewebe, natürliche oder synthetische Fasern, oder andere Schäume bevorzugt. Weiterhin ist es erfindungsgemäß bevorzugt, dass der Verbund mindestens einen Bereich beinhaltet, welcher die erfindungsgemäße superabsorbierende Zusammensetzung in einer Menge in einem Bereich von etwa 15 bis 100 Gew.-%, vorzugsweise etwa 30 bis 100 Gew.-%, besonders bevorzugt von etwa 50 bis 99,99 Gew.-%, ferner bevorzugt von etwa 60 bis 99,99 Gew.-% und darüber hinaus bevorzugt von etwa 70 bis 99 Gew.-%, jeweils bezogen auf das Gesamtgewicht des betreffenden Bereichs des Verbundes, beinhaltet, wobei dieser Bereich vorzugsweise eine Größe von mindestens 0,01 cm³, vorzugsweise mindestens 0,1 cm³ und am meisten bevorzugt mindestens 0,5 cm³ aufweist.

In einer Ausgestaltung des erfindungsgemäßen Verbundes handelt es sich um einen flächenförmigen Verbund, wie er in der WO 02/056812 A1 als "absorbent material" beschrieben ist.

Einen weiteren Beitrag zur Lösung der eingangs genannten Aufgaben liefert ein Verfahren zur Herstellung eines Verbundes, wobei die erfindungsgemäßen superabsorbierende Zusammensetzung und ein Substrat und gegebenenfalls ein Zusatzstoff miteinander in Kontakt gebracht werden. Als Substrate werden vorzugsweise diejenigen Substrate eingesetzt, die bereits vorstehend im Zusammenhang mit dem erfindungsgemäßen Verbund genannt wurden.

Einen Beitrag zur Lösung der eingangs genannten Aufgaben liefert auch ein Verbund erhältlich nach dem vorstehend beschriebenen Verfahren, wobei dieser Verbund vorzugsweise die gleichen Eigenschaften aufweist wie der vorstehend beschriebene, erfindungsgemäße Verbund.

Nach einem anderen Aspekt der vorliegenden Erfindung ist der Verbund als ein Hygieneartikelcore ausgebildet, das, jeweils bezogen auf das Hygieneartikelcore mindestens 30 Gew.- %, vorzugsweise mindestens 50 Gew.-% und darüber hinaus bevorzugt mindestens 70 Gew.- % der erfindungsgemäßen wasserabsorbierenden Zusammensetzung und mindestens 1 Gew.-%, vorzugsweise mindestens 5 Gew.-% und darüber hinaus bevorzugt mindestens 10 Gew.- % des Substrats und gegebenenfalls noch weitere übliche Hilfs- und/oder Haftmittel beinhaltet, wobei die Summe der Gewichtsprozente der einzelnen im Hygieneartikelcore beinhalteten Komponenten an 100 Gew.-% ergibt. Als Substrat im Zusammenhang mit dem Hygieneartikelcore sind besonders Materialien bevorzugt, die zur Fixierung der meist als Teilchen vorliegenden erfindungsgemäßen superabsorbierenden Zusammensetzung dienen. Hierbei kann es sich um Fasern oder Gewirke oder Gewebe sowie Netze handeln. Es ist weiterhin möglich, dass die beispielsweise als Pulver und damit teilchenförmig vorliegende superabsorbierende Zusammensetzung durch ein Haftmittel wie einen Klebstoff mit dem Substrat verbunden ist. Gleichfalls entspricht es einer Ausgestaltung, dass das Substrat so ausgestaltet ist, dass die superabsorbierende Zusammensetzung in Ausnehmung des Substrats aufgenommen wird. Übliche, ebenfalls in dem Hygieneartikelcore einarbeitbare Hilfsmittel sind beispielsweise Stoffe, kosmetische Stoffe, die die Hautverträglichkeit erhöhen, Desinfektionsmittel, antimikrobielle Substanzen und dergleichen.

In einem weiteren Aspekt betrifft die vorliegende Erfindung Hygieneartikel, beinhaltend eine flüssigkeitsdurchlässige Oberschicht, eine flüssigkeitsundurchlässige Unterschicht und einen zwischen der Oberschicht und der Unterschicht angeordneten erfindungsgemäßen Verbund. Als Hygieneartikel kommen sowohl Damenhygieneartikel, erwachsene Inkontinenzprodukte als auch Windeln für Säuglinge, Babys und Kleinkinder in Betracht. Bevorzugt ist es, dass der Hygieneartikel ein vorstehend beschriebenes Hygieneartikelcore beinhaltet. Als flüssigkeitsdurchlässige Oberschicht kommen grundsätzlich alle dem Fachmann hierfür bekannten und geeignet erscheinenden Gewebe, Gelege und Gewirklagen, die meist aus Zellstoffen oder Zellstoffderivaten bestehen, die gegebenenfalls an Kunststoffe wie Polypropylen oder Polyethylen gebunden sind, in Betracht. Auch als flüssigkeitsundurchlässige Unterschicht werden die in der Industrie dem Fachmann geläufigen meist ebenfalls aus einem Zellstoff oder Zellstoffderivat, -gelege, -gewirk, oder -gestrick bestehenden Vliese eingesetzt, wobei diese im Allgemeinen mit einer Kunststoffschicht, meist aus Polypropylen oder Polyethylen, abgedichtet sind.

Einen weiteren Beitrag zur Lösung der eingangs genannten Aufgaben liefern chemische Produkte beinhaltend die erfindungsgemäßen superabsorbierenden Zusammensetzungen oder einen erfindungsgemäßen Verbund. Bevorzugte chemische Produkte sind insbesondere Schäume, Formkörper, Fasern, Folien, Filme, Kabel, Dichtungsmaterialien, flüssigkeitsaufnehmende Hygieneartikel, insbesondere Windeln und Damenbinden, Träger für pflanzen- oder pilzwachstumsregulierende Mittel oder Pflanzenschutzwirkstoffe, Zusätze für Baustoffe, Verpackungsmaterialien oder Bodenzusätze.

Auch die Verwendung der erfindungsgemäßen Polymergebilde oder des erfindungsgemäßen Verbundes in chemischen Produkten, vorzugsweise in den vorstehend genannten chemischen Produkten, insbesondere in Hygieneartikeln wie Windeln oder Damenbinden, sowie die Verwendung der Superabsorberpartikel als Träger für pflanzen- oder pilzwachstumsregulierenden Mittel oder Pflanzenschutzwirkstoffe liefern einen Beitrag zur Lösung der eingangs genannten Aufgaben. Bei der Verwendung als Träger für pflanzen- oder pilzwachstumsregulierende Mittel oder Pflanzenschutzwirkstoffe ist es bevorzugt, dass die pflanzen- oder pilzwachstumsregulierende Mittel oder Pflanzenschutzwirkstoffe über einen durch den Träger kontrollierten Zeitraum abgegeben werden können.

Die Erfindung wird nachfolgend anhand von nichtlimitierenden Beispielen erläutert.

### Testmethoden

### Allgemein

Sofern nicht nachfolgend andere Testmethoden vorgegeben werden, wird auf die dem Fachmann allgemein bekannten und üblich erscheinenden Testmethoden zurückgegriffen, wobei insbesondere Testmethoden der EDANA (European Diaper and Nonwoven Association) Anwendung finden, die allgemein als "ERT-Methoden" angegeben werden.

### Retention

Die Retention wurde als CRC (Centrifuge Retention Capacity) nach ERT 441-2-02 an der gesamten Kornfraktion bestimmt, wobei 30 Minuten aufgesogen und direkt danach für 3 Minuten geschleudert wurde.

### Absorption

Die Absorption unter Druck wurde als AAP (Absorption at Pressure) nach ERT 442-2-02 an der gesamten Kornfraktion bestimmt. Die Tiefgestellten Zahlenangaben 0.3 und 0,7 geben die jeweiligen Druckbelastungen in psi an, unter denen die Absorption gemessen wurde. Die FA (freie Absorption) wurde nach ERT 442-2-02 an der gesamten Kornfraktion bestimmt, wobei in Abweichung zu der dort angegebenen Methode kein Druck durch Weglassen entsprechender Gewichte ausgeübt wurde.

### Permeabilität

Die Permeabilität wurde als SFC (Saline Flow Conductivity) nach EP 0 752 892 B1 an der gesamten Kornfraktion bestimmt.

### Akquisitionszeit und Flüssigkeitsverteilung

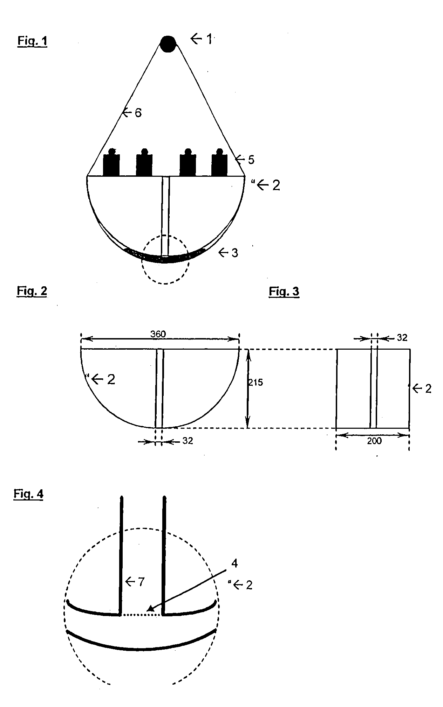

Die Messeinrichtung zur Bestimmung von Akquisitionszeit und Flüssigkeitsverteilung des Cores ist in den Figuren 1 bis 4 schematisch dargestellt. Fig. 1 zeigt einen seitlichen Schnitt durch die Messeinrichtung, wobei an einer als Laborstab ausgebildeten Aufhängung 1 über eine 200 mm breite ca. 1 mm starke, nicht elastische und glatte Kunststofffolie 6 ein aus transparentem Plexiglas gebildeter einem Gesäß eines Hygieneartikelträgers im Prinzip nachempfundener Einsatz 2 gehalten wird. Der Einsatz 2 ist im wesentlichen ein Halbrund mit einer glatten Oberfläche, das in seinem Zentrum einen Kanal 7 aufweist, der, wie in Figur 4 als Ausschnitt dargestellt über ein Sieb 4 an seiner unteren Öffnung verfügt. Das Core 3 wird mit seinem sich aus der Aufsicht ergebenden Mittelpunkt unter die untere Öffnung des Kanals 7 gegeben. Anschließend wird der Einsatz 2 mit den für die Messungen vorgesehenen Gewichten 5 beschwert. Durch die obere Öffnung des Kanals 7 wird die Testflüssigkeit in die Messeinrichtung gefüllt. Die Figuren 2 und 3 geben die Maße der Messeinrichtung von Vorderansicht und der Seitenansicht in mm an.

Das Core in einer Größe von 300 mm Länge x 120 mm Breite wurde gewogen, in die körpergeformte Testapparatur gelegt und mit 9 kg belastet. Danach wurden dreimal 60 g einer 0,9%igen wässrigen Kochsalzlösung, die mit 5 ml/l Säurefuchsin Stammlösung (in 11 einer 0,9%igen Kochsalzlösung wurden 10,0g Säurefuchsin hinzugegeben und der pH-Wert der Lösung durch Zugabe von 0,3%iger wässriger NaOH auf 6,0 eingestellt) eingefärbt war, mittels Messzylinder in einem zeitlichen Abstand von 20 Minuten durch die Öffnung der Testapparatur gegeben. Mittels Stoppuhr kann die Einsickerzeit vom Zugabestart bis zum vollständigen Einsickern der Testflüssigkeit gemessen werden. Nach jeder Flüssigkeitszugabe folgt eine Wartezeit von 20 Minuten. Anschließend werden die Gewichte abgenommen. Die Flüssigkeitsverteilung wurde jeweils nach Ablauf der oben genannten 20 Minuten bestimmt. Dazu wurde die Ausbreitung der Flüssigkeit in der Länge des Cores gemessen.

### Viskosität

Die Viskosität der Stärken wurde bei 20°C mit einem Brookfieldviskosimeter in einer 6% wässrigen Lösung bestimmt.

### pH-Wert

Der pH-Wert wurde in einer 3%igen wässrigen Lösung bei 20°C bestimmt.

### Teilchengröße

Bei wasserabsorbierenden Polymeren und der superabsorbierenden Zusammensetzungen nach Siebanalyse und Auswertung gemäß ERT 420.2-02. Im Fall von Stärkeverbindungen mit einem Beckmann Coulter LS Teilchengrößenbestimmer. Die das MMPSD (Gewichtsmittel der Teilchengröße) ergibt sich aus einer Auftragung mit halblogarithmischer Skalierung bei 50% (vgl. EP 0 304 319 B1).

### Biologische Abbaubarkeit

Diese wird nach dem modifizierten Sturm-Test gemäß Anhang V zur Richtlinie 67/548/EWG für einen Zeitraum von 28 Tagen bestimmt.

### Beispiele

### Ausgangsmaterialien:

Es wurde frisch gereinigten Ausgangsmaterialien eingesetzt. Weiterhin wurden kommerziell erhältliche, nachfolgend in Tabelle 1 aufgeführte Stärkeverbindungen der Firma Roquette GmbH, Deutschland, eingesetzt.

**Tabelle 1**

| Typ | FA [g/g] | CRC [g/g] | AAP_{0,3} [g/g] | AAP_{0,7} [g/g] | Viskosität [mPa s] | Teilchen-größe [µm] | | | pH-Wert |
|---|---|---|---|---|---|---|---|---|---|
| | | | | | | <10 | MMPSD | >100 | |
| 1 | 18,7 | 13,8 | 5,0 | 5,4 | 30.000 | | | 0% | 9-11,5 |
| 2 | 1,3 | 0,8 | | | | | | 0% | 5-7 |
| 3 | 5,7 | 5,2 | | | | | | 0% | 5-7 |
| 4 | 4,1 | 3,5 | | | | | | 0% | 5-7 |

| | | | | | | | | | |
|---|---|---|---|---|---|---|---|---|---|
| Typ 1 = Wasserlösliche carboxymethylierte Kartoffelstärke mit Handelsbezeichnung Tackidex^{®} Q 106 Typ 2 = Wasserlösliche Maisstärke mit einem Amylosegehalt von 70% mit der Handelsbezeichnung Eurylon^{®} 7 Typ 3 = Wasserlösliche Weizenstärke mit Handelsbezeichnung Floralys^{®} 380 Typ 4 = Wasserlösliche Maisstärke mit Handelsbezeichnung Tackidex^{®} 009 | | | | | | | | | |

### Beispiel 1:

### (Herstellung eines nicht nachvernetzten wasserabsorbierenden Polymergebildes als Hydrogel - Probe a)

Eine Monomerenlösung bestehend aus 300 g Acrylsäure, die zu 72 Mol-% mit 50%iger Natronlauge neutralisiert wurde, die sich aus der Angabe "WS" für die ergebende in Tabelle 2 Menge an Wasser, 0,38g Polyethylenglykol-300-diacrylat und 1,14 g Allyloxypolyethylenglykolacrylsäureester wird durch Spülen mit Stickstoff vom gelösten Sauerstoff befreit und auf die Starttemperatur von 4°C abgekühlt. Nach Zugabe der in Tabelle 2 angegebenen Stärkeverbindung, durchmischen und Erreichen der Starttemperatur wurde die Initiatorlösung (0,3 g Natriumperoxydisulfat in 10 g H₂O, 0,07 g 30%ge Wasserstoffperoxidlösung in 1 g H₂O und 0,015 g Ascorbinsäure in 2 g H₂O) zugesetzt. Nachdem die Endtemperatur von ca. 100°C erreicht war, wurde das entstandene Gel gewölft, so dass ein Granulat mit etwa 1 bis 3mm großen Teilchen erhalten wurde, die in Tabelle 2a als Proben a1 bis a4 angegeben sind. Der Wassergehalt der nichtgetrockneten Hydrogele lag bei 45 bis 65%.

**Tabelle 2a**

| Beispiel | Stärkeverbindung | Menge Stärkeverbindung [%] | WS - Menge Acrylsäure [% in Monomerlsg.] | Probe |
|---|---|---|---|---|
| 1a | Typ 1 | 5 | 30 - 441,65g Wasser | a1 |
| 1b | Typ 1 | 5 | 27,5 - 486,69g Wasser | a2 |
| 1c | Typ 1 | 5 | 25 - 531,82g Wasser | a3 |
| 1d (vgl.) | keine | 0 | 30 - 441,65g Wasser | a4 |

### Beispiele 1e-1 bis 1e-24 (erfindungsgemäß)

### (Einarbeiten von Stärkeverbindungen in Hydrogel - Probe a zu Proben V2)

Das in Beispiel 1 ohne Stärkeverbindung erhaltene Hydrogel wurde vor einem weiteren Wölfen mit in Tabelle 2b angegebenen Mengen Stärkeverbindung versetzt und gemäß den Vorschriften der Beispiele 2a und 3a getrocknet, abgesiebt und nachvernetzt.

**Tabelle 2b**

| Stärke Typ | 1 | 2 | 3 | 4 |
|---|---|---|---|---|
| Menge | Proben | Proben | Proben | Proben |
| 5% | V2-1a | V2-2a | V2-3a | V2-4a |
| 10% | V2-1b | V2-2b | V2-3b | V2-4b |
| 20% | V2-1c | V2-2c | V2-3c | V2-4c |

### Beispiel 2:

### (Herstellung eines nicht nachvernetzten wasserabsorbierenden Polymergebildes als Pulver - Probe b)

Probe a1 bis a4 wurde bei 150°C 120 Minuten lang getrocknet. Das getrocknete Polymerisat wurde grob zerstoßen, gemahlen und auf ein Pulver mit einer Partikelgröße von 150 bis 850 µm gesiebt und Proben b1 bis b4 mit einer mittleren Teilchengröße nach ERT 420.2-02 von 520 bis 540 µm erhalten. Der nach ERT 430.1-99 bestimmte Wassergehalt betrug bei allen Proben b1 bis b4 5%. Weitere Eigenschaften ergeben sich aus Tabelle 3a.

**Tabelle 3a**

| Beispiel | 2a | 2b | 2c | 2d(vgl.) |
|---|---|---|---|---|
| Probe | b1 | b2 | b3 | b4 |
| CRC [g/g] | 32,1 | 32,5 | 34,6 | 35,1 |

### Beispiele 2e-1 bis 2e-24

### (Einarbeiten von Stärkeverbindungen auf pulverförmiges nicht nachvernetztes wasserabsorbierendes Polymergebilde - Probe a zu Proben V4)

Die in Beispiel 2 erhalten Probe b4 wurde in einem Rollenmixer Typ BTR 10 der Firma Fröbel GmbH, Deutschland mit in Tabelle 3b angegebenen Mengen Stärkeverbindung innig vermischt und gemäß der Vorschrift des Beispiels 3a nachvernetzt.

**Tabelle 3b**

| Stärke Typ | 1 | 2 | 3 | 4 |
|---|---|---|---|---|
| Menge | Proben | Proben | Proben | Proben |
| 5% | V4-1a | V4-2a | V4-3a | V4-4a |
| 10% | V4-1b | V4-2b | V4-3b | V4-4b |
| 20% | V4-1c | V4-2c | V4-3c | V4-4c |

### Beispiel 3a:

### (Herstellung eines nachvernetzten wasserabsorbierenden Polymergebildes als Pulver - Probe c)

Zur Nachvernetzung wurden 100g des oben erhaltenen Pulvers der Proben b1 bis b4 unter kräftigen Rühren mit einer Lösung aus 1 g 1,3-Dioxalan-2-on, 3g Wasser vermischt und anschließend für 30 Minuten in einem Ofen, der auf 180°C temperiert war, erhitzt. Die so erhaltenen Proben c1 bis c4 hatten eine mittlere Teilchengröße nach ERT 420.2-02 von 525 bis 573 µm. Der in Probe c nach ERT 430.1-99 bestimmte Wassergehalt betrug 4,5%. Einzelheiten ergeben sich aus Tabelle 4a.

**Tabelle 4a**

| Beispiel | 3a | 3b | 3c | 3d(vgl.) |
|---|---|---|---|---|
| Probe | c1 | c2 | c3 | c4 |
| AAP_{0,7} [g/g] | 23,6 | 24,4 | 25,3 | 25,0 |
| CRC [g/g] | 27,2 | 27,3 | 29,2 | 30,6 |
| SFC [*10⁻⁷ cm³ sec/g] | | | 29,2 | 20,0 |

### Beispiele 3e-1 bis 3e-24

### (Einarbeiten von Stärkeverbindungen auf pulverförmiges nachvernetztes wasserabsorbierendes Polymergebilde - Probe a zu Proben V6)

Die in Beispiel 3a erhalten Probe c4 wurde in einem Rollenmixer Typ BTR 10 der Firma Fröbel GmbH, Deutschland mit in Tabelle 4b angegebenen Mengen Stärkeverbindung innig vermischt.

**Tabelle 4b**

| Stärke Typ | 1 | 2 | 3 | 4 |
|---|---|---|---|---|
| Menge | Proben | Proben | Proben | Proben |
| 5% | V6-1a | V6-2a | V6-3a | V6-4a |
| 10% | V6-1b | V6-2b | V6-3b | V6-4b |
| 20% | V6-1c | V6-2c | V6-3c | V6-4c |

### Beispiele 3e-25 bis 3e-48

### (Einarbeiten von Stärkeverbindungen auf pulverförmiges nachvernetztes wasserabsorbierendes, Stärkeverbindungen beinhaltendes Polymergebilde - Probe a zu Proben V1V6)

Die in Beispiel 3a erhalten Probe c3 wurde in einem Rollenmixer Typ BTR 10 der Firma Fröbel GmbH, Deutschland mit in Tabelle 4c angegebenen Mengen Stärkeverbindung innig vermischt.

**Tabelle 4c**

| Stärke Typ | 1 | 2 | 3 | 4 |
|---|---|---|---|---|
| Menge | Proben | Proben | Proben | Proben |
| 5% | V1V6-1a | V1V6-2a | V1V6-3a | V1V6-4a |
| 10% | V1V6-1b | V1V6-2b | V1V6-3b | V1V6-4b |
| 20% | V1V6-1c | V1V6-2c | V1V6-3c | V1V6-4c |

### Beispiele 4.1 zu V2V6-Proben (erfindungsgemäß)

### (Einarbeiten von Stärkeverbindungen auf pulverförmiges nachvernetztes wasserabsorbierendes, Stärkeverbindungen beinhaltendes Polymergebilde - Probe a zu Proben V2V6)

Die Proben V2-1a bis V2-4c wurde in einem Rollenmixer Typ BTR 10 der Firma Fröbel GmbH, Deutschland mit den Stärkeverbindung der Typen 1 bis 4 jeweils in Stärkeverbindungsmengen von 5%, 10% und 20% innig vermischt.

### Beispiele 4.2 zu V4V6-Proben

### (Einarbeiten von Stärkeverbindungen auf pulverförmiges nachvernetztes wasserabsorbierendes Polymergebilde - Probe a zu Proben V4V6)

Die Proben V4-1a bis V4-4c wurde in einem Rollenmixer Typ BTR 10 der Firma Fröbel GmbH, Deutschland mit den Stärkeverbindung der Typen 1 bis 4 jeweils in Stärkeverbindungsmengen von 5%, 10% und 20% innig vermischt.

### Beispiel 4: Polymerisation

Eine Monomerenlösung bestehend aus 280 g der vorstehend gewonnenen Acrylsäure, die zu 70 Mol-% mit Natronlauge neutralisiert wurde, 466,8 g Wasser, 1,4 g Polyethylenglykol-300-diacrylat und 1,68 g Allyloxypolyethylenglykolacrylsäureester wird durch Spülen mit Stickstoff vom gelösten Sauerstoff befreit und auf die Starttemperatur von 4°C abgekühlt. Nach Erreichen der Starttemperatur wurde die Initiatorlösung (0,1 g 2,2'-Azobis-2-amidinpropan-dihydrochlorid in 10 g H₂O, 0,3 g Natriumperoxydisulfat in 10 g H₂O, 0,07 g 30%ge Wasserstoffperoxidlösung in 1 g H₂O und 0,015 g Ascorbinsäure in 2 g H₂O) zugesetzt. Nachdem die Endtemperatur von ca. 100°C erreicht war, wurde das entstandene Gel zerkleinert und bei 150°C 90 Minuten lang getrocknet. Das getrocknete Polymerisat wurde grob zerstoßen, gemahlen und auf ein Pulver A mit einer Partikelgröße von 150 bis 850 µm gesiebt.

Zur Nachvernetzung wurden 100g des oben erhaltenen Pulvers A unter kräftigen Rühren mit einer Lösung aus 1 g 1,3-Dioxalan-2-on, 3g Wasser und 0,5 g Aluminiumsulfat-18-Hydrat vermischt und anschließend für 40 min in einem Ofen, der auf 180°C temperiert war, erhitzt. Das so erhaltene Pulver B hat eine mittlere Teilchengröße nach ERT 420.2-02 von 530 µm. Der in Pulver A nach ERT 430.1-99 bestimmte Wassergehalt betrug 4,5%.

Das Pulver B wurde mit einer Stärkeverbindung des Typs 3 im Gewichtsverhältnis Polymer: Stärke von 4 : 1 unter trockenen Bedingungen gemischt und anschließend für 45 Minuten in einem Rollenmixer Typ BTR 10 der Firma Fröbel GmbH, Deutschland, weiter homogenisiert.

Das Pulver C wies eine biologische Abbaubarkeit gemäß dem modifizierten Sturmtest nach 28 Tagen von 39 % und einen CRC-Wert von 29,9 g/g auf.

### Core-Herstellung

Die Cores wurden mittels einer Mischung von jeweils 48,6 Gew.-% der vorstehend dargestellten superabsorbierenden Zusammensetzungen V6-2b bzw. V6-2c bezogen auf das Core, und 48,6 Gew.-% Zellulosefasern Stora Fluff EF semitreated der Firma Stora-Enzo AB Schweden, sowie 2,8 Gew.-% einer Zweikomponentenfaser aus jeweils 50 Gew.-% Polypropylen (PP) bzw. Polyethylen (PE) mit PP Kern und PE Mantel der Firma Fibervision A/S Dänemark durch ein Airlaid-Verfahren mit einer M&J-Maschine (Breite 40cm, Arbeitsbreite 36cm, Betriebseinstellungen: Bandgeschwindigkeit 3 m/min, Fluffeinzug an Hammermühle 3,1 m/min, Polymerdosierung 380 g/min, Zweikomponentenfaser in 10g Portionen ca. 1 mal/min abgeworfen) gebildet, wobei das absorbierende Polymer homogen eingetragen wurde. Cores mit einem Basisgewicht von 756 g/m², eine Dichte von 0,2 g/cm³ wurden für die Prüfungen verwendet. Die Cores hatte ein Länge von 300 mm und eine Breite von 120 mm. Für die jeweiligen als "Insults" bezeichneten Flüssigkeitsgaben sind die Ergebnisse der Core-Analyse sind in Tabelle 5a und 5b dargestellt.

**Tabelle 5a**

| Aquisitionszeit als 6-Fachbestimmung | | | |
|---|---|---|---|
| Probe | 1. Insult | 2. Insult | 3. Insult |
| V6-2b | 22s | 200s | 562s |
| V6-2c | 20s | 167s | 468s |
| 3d (vgl.) | 23s | 290s | 797s |

**Tabelle 5b**

| Flüssigkeitsverteilung als 6-Fachbestimmung | | | |
|---|---|---|---|
| Probe | 1. Insult | 2. Insult | 3. Insult |
| V6-2b | 10cm | 15cm | 19,8cm |
| V6-2c | 10cm | 16cm | 20,3cm |
| 3d (vgl.) | 10cm | 14cm | 18,2cm |

Die superabsorbierenden Zusammensetzungen zeigen eine verbesserte Aquisitionszeit und Flüssigkeitsverteilungen gegenüber einer nicht eine Stärkeverbindung enthaltenden Vergleichsprobe.

## Patentansprüche

1. Verfahren zur Herstellung einer superabsorbierenden Zusammensetzung, beinhaltend als Verfahrensschritte
i. Herstellen eines Hydrogels durch radikalische Polymerisation einer wässrigen, mindestens ein Monomer beinhaltende Monomerlösung;
ii. Trocknen des Hydrogels unter Erhalt eines wasserabsorbierenden Polymergebildes;
iii. Oberflächenvernetzung des wasserabsorbierenden Polymergebildes unter Erhalt eines oberflächenvernetzten wasserabsorbierenden Polymergebildes;
iv. Einarbeiten einer Stärkeverbindung nach Verfahrensschritt i. und vor Verfahrensschritt ii. in das Hydrogel.

2. Verfahren nach Anspruch 1, umfassend Einarbeiten einer Stärkeverbindung nach Verfahrensschritt iii auf das oberflächenvernetzte wasserabsorbierende Polymergebilde.

3. Verfahren nach Anspruch 1 oder 2, wobei die Stärkeverbindung wasserlöslich ist.

4. Verfahren nach einem der Ansprüche 1 bis 3, wobei mindestens 70 Gew.-% der Stärkeverbindung eine Teilchengröße von kleiner 100 µm aufweisen.

5. Verfahren nach einem der vorhergehenden Ansprüchen, wobei die Stärkeverbindung mindestens eine der folgenden Eigenschaften besitzt
S1 eine freie Absorption (FA) von mindestens 9 g/g;
S2 eine Retention (CRC) von mindestens 7 g/g;
S3 eine Absorption gegen Druck (AAP_{0,3}) von mindestens 2g/g;
S4 eine Absorption gegen Druck (AAP_{0,7}) von mindestens 2g/g;
S5 einen pH-Wert im Bereich von 9 bis 14.

6. Verfahren nach einem der vorhergehenden Ansprüche, wobei mindestens eine erste Stärkeverbindung SI nach Verfahrensschritt i. und vor Verfahrensschritt ii. in das Hydrogel eingearbeitet wird.

7. Verfahren nach Anspruch 6, wobei zusätzlich zu der mindestens einen ersten Stärkeverbindung SI mindestens eine weitere Stärkeverbindung SII nach Verfahrensschritt iii auf das oberflächenvernetzte wasserabsorbierende Polymergebilde eingearbeitet wird.

8. Verfahren nach Anspruch 6 oder 7, wobei sich die erste Stärkeverbindung SI und die weitere Stärkeverbindung SII in mindestens einem Merkmal voneinander unterscheiden.

9. Verfahren nach Anspruch 8, wobei die sich die erste Stärkeverbindung SI und die weitere Stärkeverbindung SII in mindestens einem der folgenden Merkmale voneinander unterscheiden
D1 in der freien Absorption (FA);
D2 in der Retention (CRC);
D3 in dem pH-Wert;
D4 in der chemischen Zusammensetzung.

10. Verfahren nach einem der Ansprüche 7 bis 9, wobei die weitere Stärkeverbindung SII mindestens eine der folgenden Merkmale aufweist
d1 eine freie Absorption (FA) von weniger als 9 g/g;
d2 eine Retention (CRC) von weniger als 7 g/g;
d3 einen pH-Wert in einem Bereich von 4 bis <9.

11. Verfahren nach einem der vorhergehenden Ansprüche, wobei die Stärkeverbindung in einer Menge von maximal 30 Gew.-%, bezogen auf das eingesetzte Monomer, eingesetzt wird.

12. Verfahren nach einem der vorhergehenden Ansprüche, wobei die Monomerlösung einen Monomergehalt im Bereich von 15 bis 35 Gew.-%, bezogen auf die Monomerlösung, hat.

13. Verfahren nach einem der vorhergehenden Ansprüche, wobei die Stärkeverbindung eine Lösungsviskosität nach der hier angegebenen Methode im Bereich 5.000 bis 300.000 mPas hat.

14. Verfahren nach einem der vorhergehenden Ansprüche, wobei die Stärkeverbindung und die erste Stärkeverbindung SI eine carboxyalkylierte Stärkeverbindung ist.

15. Verfahren nach einem der vorhergehenden Ansprüche, wobei die Stärkeverbindung weniger als 10 Gew.-% zyklische oder verzweigte Polysaccharide aufweist.

16. Verfahren nach einem der vorhergehenden Ansprüche, wobei die superabsorbierende Zusammensetzung mindestens eine der folgenden Eigenschaften besitzt
Z1 eine Retention (CRC) von mindestens 25 g/g;
Z2 eine Absorption gegen Druck (AAP_{0,7}) von mindestens 15g/g;
Z3 einen Permeabilität (SFC) von mindestens 20* 10⁻⁷ cm³ sec/g
Z4 eine gemäß der hier angegebnen Methode nach 28 Tagen von mindestens 25 %.

17. Eine superabsorbierende Zusammensetzung, erhältlich nach einem Verfahren nach einem der vorhergehenden Ansprüche.

18. Verbund, beinhaltend eine superabsorbierende Zusammensetzung nach Anspruch 17 und ein Substrat.

19. Verbund nach Anspruch 18 als Hygineartikelcore ausgebildet, beinhaltend, jeweils bezogen auf das Hygieneartikelcore, mindestens 30 Gew.-% der superabsorbierenden Zusammensetzung nach Anspruch 17 und mindestens 1 Gew.-% des Substrats.

20. Hygieneartikel, beinhaltend eine flüssigkeitsdurchlässige Oberschicht, eine flüssigkeitsundurchlässige Unterschicht und einen zwischen der Oberschicht und der Unterschicht angeordneten Verbund nach einem der Ansprüche 18 oder 19.

21. Schäume, Formkörper, Fasern, Folien, Filme, Kabel, Dichtungsmaterialien, flüssigkeitsaufnehmende Hygieneartikel, Träger für pflanzen- und pilzwachstumregulierende Mittel, Verpackungsmaterialien, Bodenzusätze oder Baustoffe, beinhaltend eine superabsorbierende Zusammensetzung nach Anspruch 17 oder einen Verbund nach einem der Ansprüche 18 oder 19.

22. Verwendung einer superabsorbierenden Zusammensetzung nach Anspruch 17 oder eines Verbunds nach einem der Ansprüche 18 oder 19 in Schäumen, Formkörpern, Fasern, Folien, Filmen, Kabeln, Dichtungsmaterialien, flüssigkeitsaufnehmenden Hygieneartikeln, Trägern für pflanzen- und pilzwachstumregulierende Mittel, Verpackungsmaterialien, Bodenzusätzen oder Baustoffen.

## Claims

1. Process for producing a superabsorbent composition, including as process steps
i. preparing a hydrogel by radical polymerization of an aqueous monomer solution including at least one monomer;
ii. drying the hydrogel to give a waterabsorbing polymer structure;
iii. surface-crosslinking the water-absorbing polymer structure to give a surface-crosslinked water-absorbing polymer structure;
iv. incorporating a starch compound into the hydrogel after process step i. and before process step ii.

2. Process according to Claim 1, comprising incorporating a starch compound onto the surface-crosslinked water-absorbing polymer structure after process step iii.

3. Process according to Claim 1 or 2, where the starch compound is water-soluble.

4. Process according to any of Claims 1 to 3, where at least 70 wt% of the starch compound has a particle size of less than 100 µm.

5. Process according to any of the preceding claims, where the starch compound possesses at least one of the following properties
S1 a free absorption (FA) of at least 9 g/g;
S2 a retention (CRC) of at least 7 g/g;
S3 an absorption against pressure (AAP_{0.3}) of at least 2 g/g;
S4 an absorption against pressure (AAP_{0.7}) of at least 2 g/g;
S5 a pH in the range from 9 to 14.

6. Process according to any of the preceding claims, where at least one first starch compound SI is incorporated into the hydrogel after process step i. and before process step ii.

7. Process according to Claim 6, where additionally to the at least one first starch compound SI, at least one further starch compound SII is incorporated onto the surface-crosslinked waterabsorbing polymer structure after process step iii.

8. Process according to Claim 6 or 7, where the first starch compound SI and the further starch compound SII differ from one another in at least one feature.

9. Process according to Claim 8, where the first starch compound SI and the further starch compound SII differ from one another in at least one of the following features
D1 in the free absorption (FA);
D2 in the retention (CRC);
D3 in the pH;
D4 in the chemical composition.

10. Process according to any of Claims 7 to 9, where the further starch compound SII has at least one of the following features
d1 a free absorption (FA) of less than 9 g/g;
d2 a retention (CRC) of less than 7 g/g;
d3 a pH in a range from 4 to < 9.

11. Process according to any of the preceding claims, where the starch compound is used in an amount of at most 30 wt%, based on the monomer used.

12. Process according to any of the preceding claims, where the monomer solution has a monomer content in the range from 15 to 35 wt%, based on the monomer solution.

13. Process according to any of the preceding claims, where the starch compound has a solution viscosity according to the method specified here in the 5000 to 300 000 mPas range.

14. Process according to any of the preceding claims, where the starch compound and the first starch compound SI is a carboxyalkylated starch compound.

15. Process according to any of the preceding claims, where the starch compound has less than 10 wt% of cyclic or branched polysaccharides.

16. Process according to any of the preceding claims, where the superabsorbent composition possesses at least one of the following properties
Z1 a retention (CRC) of at least 25 g/g;
Z2 an absorption against pressure (AAP_{0.7}) of at least 15 g/g;
Z3 a permeability (SFC) of at least 20*10⁻⁷ cm³ sec/g
Z4 a after 28 days according to the method specified here of at least 25%.

17. A superabsorbent composition obtainable by a process according to any of the preceding claims.

18. Assembly including a superabsorbent composition according to Claim 17 and a substrate.

19. Assembly according to Claim 18 in the form of a hygiene article core, including, based in each case on the hygiene article core, at least 30 wt% of the superabsorbent composition according to Claim 17 and at least 1 wt% of the substrate.

20. Hygiene article including a liquid-pervious top layer, a liquid-impervious bottom layer and, disposed between the top layer and the bottom layer, an assembly according to either of Claims 18 and 19.

21. Foams, mouldings, fibres, foils, films, cables, sealing materials, liquid-absorbing hygiene articles, carriers for plant growth and fungal growth regulators, packaging materials, soil additives or building materials, including a superabsorbent composition according to Claim 17 or an assembly according to either of Claims 18 and 19.

22. Use of a superabsorbent composition according to Claim 17 or of an assembly according to either of Claims 18 and 19 in foams, mouldings, fibres, foils, films, cables, sealing materials, liquid-absorbing hygiene articles, carriers for plant growth and fungal growth regulators, packaging materials, soil additives or building materials.

## Revendications

1. Procédé de fabrication d'une composition superabsorbante, comprenant en tant qu'étapes de procédé :
i. la fabrication d'un hydrogel par polymérisation radicalaire d'une solution aqueuse de monomères contenant au moins un monomère ;
ii. le séchage de l'hydrogel pour obtenir une structure polymère absorbant l'eau ;
iii. la réticulation de surface de la structure polymère absorbant l'eau pour obtenir une structure polymère absorbant l'eau réticulée en surface ;
iv. l'incorporation d'un composé d'amidon dans l'hydrogel après l'étape de procédé i. et avant l'étape de procédé ii.

2. Procédé selon la revendication 1, comprenant l'incorporation d'un composé d'amidon après l'étape de procédé iii sur la structure polymère absorbant l'eau réticulée en surface.

3. Procédé selon la revendication 1 ou 2, dans lequel le composé d'amidon est soluble dans l'eau.

4. Procédé selon l'une quelconque des revendications 1 à 3, dans lequel au moins 70 % en poids du composé d'amidon présente une taille de particule inférieure à 100 µm.

5. Procédé selon l'une quelconque des revendications précédentes, dans lequel le composé d'amidon présente au moins une des propriétés suivantes :
S1 une absorption libre (FA) d'au moins 9 g/g ;
S2 une rétention (CRC) d'au moins 7 g/g ;
S3 une absorption sous pression (AAP_{0,3}) d'au moins 2 g/g ;
S4 une absorption sous pression (AAP_{0,7}) d'au moins 2 g/g ;
S5 un pH dans la plage allant de 9 à 14.

6. Procédé selon l'une quelconque des revendications précédentes, dans lequel au moins un premier composé d'amidon SI est incorporé dans l'hydrogel après l'étape de procédé i. et avant l'étape de procédé ii.

7. Procédé selon la revendication 6, dans lequel, en plus dudit au moins un premier composé d'amidon SI, au moins un autre composé d'amidon SII est incorporé sur la structure polymère absorbant l'eau réticulée en surface après l'étape de procédé iii.

8. Procédé selon la revendication 6 ou 7, dans lequel le premier composé d'amidon SI et l'autre composé d'amidon SII diffèrent l'un de l'autre au niveau d'au moins une caractéristique.

9. Procédé selon la revendication 8, dans lequel le premier composé d'amidon SI et l'autre composé d'amidon SII diffèrent l'un de l'autre au niveau d'au moins une des caractéristiques suivantes :
D1 l'absorption libre (FA) ;
D2 la rétention (CRC) ;
D3 le pH :
D4 la composition chimique.

10. Procédé selon l'une quelconque des revendications 7 à 9, dans lequel l'autre composé d'amidon SII
d1 une absorption libre (FA) inférieure à 9 g/g ;
d2 une rétention (CRC) inférieure à 7 g/g ;
d3 un pH dans une plage allant de 4 à < 9.

11. Procédé selon l'une quelconque des revendications précédentes, dans lequel le composé d'amidon est utilisé en une quantité d'au plus 30 % en poids, par rapport au monomère utilisé.

12. Procédé selon l'une quelconque des revendications précédentes, dans lequel la solution de monomères a une teneur en monomères dans la plage allant de 15 à 35 % en poids, par rapport à la solution de monomères.

13. Procédé selon l'une quelconque des revendications précédentes, dans lequel le composé d'amidon a une viscosité en solution selon la méthode indiquée ici dans la plage allant de 5 000 à 300 000 mPas.

14. Procédé selon l'une quelconque des revendications précédentes, dans lequel le composé d'amidon et le premier composé d'amidon SI sont un composé d'amidon carboxyalkylé.

15. Procédé selon l'une quelconque des revendications précédentes, dans lequel le composé d'amidon comprend moins de 10 % en poids de polysaccharides cycliques ou ramifiés.

16. Procédé selon l'une quelconque des revendications précédentes, dans lequel la composition superabsorbante présente au moins une des propriétés suivantes :
Z1 une rétention (CRC) d'au moins 25 g/g ;
Z2 une absorption sous pression (AAP_{0,7}) d'au moins 15 g/g ;
Z3 une perméabilité (SFC) d'au moins 20*10⁻⁷ cm³s/g ;
Z4 une après 28 jours selon la méthode indiquée ici d'au moins 25 %.

17. Composition superabsorbante, pouvant être obtenue par un procédé selon l'une quelconque des revendications précédentes.

18. Composite, contenant une composition superabsorbante selon la revendication 17 et un substrat.

19. Composite selon la revendication 18, configuré sous la forme d'un noyau d'article d'hygiène, contenant, à chaque fois par rapport au noyau d'article d'hygiène, au moins 30 % en poids de la composition superabsorbante selon la revendication 17 et au moins 1 % en poids du substrat.

20. Article d'hygiène, contenant une couche supérieure perméable aux liquides, une couche inférieure imperméable aux liquides et un composite selon l'une quelconque des revendications 18 ou 19 agencé entre la couche supérieure et la couche inférieure.

21. Mousses, corps moulés, fibres, feuilles, films, câbles, matériaux d'étanchéité, articles d'hygiène absorbant les liquides, supports pour agents de régulation de la croissance de plantes et de champignons, matériaux d'emballage, amendements pour le sol ou matériaux de construction, contenant une composition superabsorbante selon la revendication 17 ou un composite selon l'une quelconque des revendications 18 ou 19.

22. Utilisation d'une composition superabsorbante selon la revendication 17 ou un composite selon l'une quelconque des revendications 18 ou 19 dans des mousses, corps moulés, fibres, feuilles, films, câbles, matériaux d'étanchéité, articles d'hygiène absorbant les liquides, supports pour agents de régulation de la croissance de plantes et de champignons, matériaux d'emballage, amendements pour le sol ou matériaux de construction.
